# EUROPEAN PATENT SPECIFICATION

(11) **EP 0 696 460 B1**
(45) Date of publication and mention of the grant of the patent: **08.12.1999**
(21) Application number: 95305337.8
(22) Date of filing: 31.07.1995
(51) Int. Cl.: A61M 39/26, A61M 39/02

(54) **Valved adapter for medical access devices**
Ventilanschluss für medizinische Injektionsstelle
Raccord à soupape pour site d'injection médicale

(30) Priority: 10.08.1994 US 288170
(43) Date of publication of application: 14.02.1996
(73) Proprietor: Becton, Dickinson and Company, Franklin Lakes, New Jersey 07417-1880 (US)
(72) Inventor: Orr, Douglas P., Sandy, Utah 84094 (US)
(74) Representative: Ruffles, Graham Keith

(56) References cited:
- EP-A- 0 430 398
- EP-A- 0 544 581
- EP-A- 0 629 418
- EP-A- 0 684 050
- WO-A-95/25552
- US-A- 5 006 114
- US-A- 5 085 645

## Description

### Field of Invention

The present invention relates to infusion devices and more particularly to a valved adapter useful with a variety of medical access devices.

### Background of the Invention

Medical access devices, particularly infusion devices, over-the-needle catheters, other catheters and feeding tubes are important tools for administration of fluids to patients. After placement, in normal management of a catheter or other medical access device in a patient, it is often necessary to be able to add or withdraw fluids through the device. In many surgical procedures, it is routine to place an intravenous catheter so that if it is necessary to medicate a patient during a procedure, the catheter already is in place. In other types of procedures it is necessary to periodically administer medicaments through the device or to withdraw samples. In all of these applications, the presence of a valve mechanism on the device to facilitate the addition and to close the device after the addition is desirable.

United States Patent 5,085,645 teaches an over-the-needle catheter having an integral valve in a passage in the catheter hub. The patent discloses a valve adapter that is an integral part of a catheter hub.

United States Patent 5,251,873 teaches a medical coupling site that is adapted to be attached directly to a standard male luer lock fitting. The coupling site includes a valve element contained within a tubular retainer. The coupling site includes a slit rubber diaphragm valve that is deflected by introduction of a male luer fitting and closes by the removal of the male luer. According to international standards, there is an allowable range of 2.5mm in engagement length of a luer fitting. This variation in engagement length occurs because of variation in the outside diameter of the male projection and the inside diameter of the female receptacle of the luer fittings. Thus, a "fat" male luer will result in a "short" engagement length and conversely. Valves of the type disclosed in 5,251,873 may not open fully with male luer fittings at the "short" end of the allowable dimension, and since they also depend on the diaphragm for sealing around the male luer tip, they may also leak when a male fitting is mounted or may not fully close once opened.

United States Patent 5,108,380 discloses a valve device for a hub member of a catheter. The valve is actuated by the placement of a male luer fitting which displaces a piston biased by a coil spring to open the valve.

United States Patent 5,269,771 discloses a needleless introducer with a hemostatic valve. The valve mechanism includes a plunger biased by a coil spring which, upon actuation, spreads a pair of resilient valve elements. This design may not be fully opened by a male luer fitting at the "short" side of the dimension, and the sealing depends upon the resilient valve elements closing against themselves. Further, the valve disclosed in U.S. Patent. 5,269,771 is composed of several different materials and is complex to assemble.

Valves and adapters of the type described above fall into a medical device category often referred to as "PRN" from the Latin pro re nata, i.e., as the circumstances may require. A typical example of usage for this type device might be on a catheter left in place for three days. During this three day usage duration, a bolus dosage of a medicament might be given every 4 hours using a protocol including at each dosage interval: a) flushing the catheter to check patency; b) administration of the medicament; and c) flushing the medicament from the catheter with heparin or saline. During this typical usage period, this typical protocol results in 54 operations of the valve, i.e., 6 times a day, 3 steps each time and 3 days. In between each dosage the valve must not leak, but it must be readily reopened. Previously, the bolus introduction may have been made using hypodermic needles penetrating a resilient septum. However, a septum is likely to start leaking after multiple penetrations and given the concerns about risks to practitioners and service personnel from "sharps," hospitals have changed many protocols to reduce the use of pointed hypodermic needles. The PRN adapters as described above have been developed to address the hospitals' changing needs.

While the teachings cited above address many of the practitioners' concerns, there is still a need for a valved adapter for medical access devices that offers rapid, easy-to-use access with automatic positive on/off flow control. Additionally, many of the currently available adapters are open at the proximal end requiring an additional plug or cover to avoid contamination of the fitting when a fluid delivery device is not mounted. A device having these features plus the advantage of being self closing, easily cleanable and simple to manufacture is disclosed herein below.

US 5, 006, 114 describes a medical valve assembly in which the valve assembly has a female liner lock portion on an inlet end of the device and a male liner lock portion on the outlet portion of the valve. A movable piston seals the inlet portion of the valve from the outlet portion when the piston is in the closed position. When a syringe is attached to the inlet portion of the valve assembly, the piston is displaced in a manner that unseals a fluid channel inlet. This connects the end of the syringe to the fluid channel which is connected, at its outlet end, to a catheter or other device, connected to a patient. Thus, medication can be introduced to, or fluids aspirated from, a patient's bloodstream, without the use of a syringe having a needle. When the syringe is removed from the inlet to the valve assembly, the piston is urged by a biasing means into its original closed position creating a seal between the inlet portion of the valve and the outlet portion of the valve. This feature maintains catheter patency. The top surface of the piston along with the area of the female liner lock connector does not have any fluid reservoir and can be easily cleaned to prevent the possibility of bacterial infection as a result of repeated uses of the valve assembly.

EP-A 629, 418 published 21 December 1994 and which falls for consideration under Article 54(3) EPC, relates to a needleless access device an method of using device. The needleless access device has a housing having an inlet opening and an outlet opening and a channel formed therethrough. Moveably friction fitted in the channel adjacent the inlet opening is a piston. The piston is biased upwardly by a stretchable element. The stretchable element allows the top of the piston to be depressed by a syringe to a level adjacent internal flow channels to permit injection and aspiration of fluids through the device.

PCT/WO 95/25552 published 28 September 1995 and which falls for consideration under Article 54(3) EPC, describes a medical liquid check valve which contains a spring-biased piston provided with primary and secondary seals. A sheath extending downward from the primary seal prevents venting air from mixing with the liquid. The sheath is anchored to a vent plug which, in the preferred embodiment, functions as a cross-over for the liquid and air flow paths.

EP-A 684, 050 published 29 November 1995 and which falls for consideration under Article 54(3) EPC, discloses a needleless injection site allowing infusion and withdrawal of liquid therethrough. When a compatible blunt male fluid connector fitting is inserted, fluid flow is accommodated; but upon withdrawal of the connector, the injection site is sealed against fluid flow. The injection site includes an elastomeric plunger having first and second ends and having an annular seal portion at a selected distance from the first end, movably contained within a fluid passageway through a housing having first and second ends, and biased towards a first position in sealing contact with the housing at the first end. The housing also has at least one fluid bypass opening into the passageway intermediate the ends a selected distance from the first end. Insertion of a compatible male fitting in the first end of the injection site moves the plunger to a second position where the annular seal portion of the plunger is located adjacent the fluid bypass opening. The allows fluid flow through the passageway, bypassing the seal portion through the fluid bypass.

### Summary of the Invention

According to the present invention, there is provided a valved adaptor for a medical device comprising:
a body with a longitudinal axis having a proximal first end comprising a female luer fitting having a proximal surface, a distal second end and a passageway therethrough having an inside surface, said passageway having a chamber defined by a proximal shoulder and a distal shoulder projecting inwardly from said surface of said passageway, said chamber being intermediate said first end and said second end;
a valve contained within said chamber for selectively obstructing and allowing fluid flow through said passageway, said valve comprising an elongate resilient member having an axis substantially coaxial to said longitudinal axis of said body and having a distal end and a proximal end, said resilient member being axially compressed between said proximal shoulder and said distal shoulder, said compression of said elongate resilient member alone biasing said valve to a normally closed position thereby obstructing fluid flow through said passageway; and
a pusher on said elongate resilient member for selectively further axially compressing said elongate resilient member thereby opening said valve and allowing fluid flow through said passageway, said pusher having a proximally extending portion within said female liner fitting so that when a fluid handling device having a male liner fitting is mounted on said proximal end of said adapter having said female luer fitting, the male luer fitting will engage said pusher thereby further compressing said resilient member so that fluid may flow through said passageway, said pusher proximally extending portion having a surface forming a substantially continuous surface with said female liner fitting proximal surface thereby closing said female liner fitting when said valve is in said normally closed position;
   characterised in that the elongate resilient member is formed from an elastomer, the distal shoulder is a segmented distal shoulder having openings therethrough between said segments,
   said proximal end of said resilient member forms a fluid tight seal with said proximal shoulder upon said compression of said elongate resilient member biasing said valve to a normally closed position thereby obstructing fluid flow through said passageway, and said pusher is for selectively further axially compressing said elongate resilient member so that said proximal end of said elongate resilient member no longer forms said fluid tight seal with said proximal shoulder.

The present invention is a simple to manufacture valved adapter for medical access devices. The adapter may be readily attached to medical access devices such as feeding tubes, vascular access devices, and the like. The valved adapter provides rapid access for mounting and dismounting fluid handling devices to medical access devices such as catheters. The adapter of the present invention provides positive automatic starting and stopping of fluid flow through the device with mounting and dismounting of fluid handling devices onto medical access devices. The adapter of the present invention minimizes exposure of medical practitioners to patient body fluids, eliminates the need for "sharps" to connect to medical access devices and minimizes the chance of air introduction to the patient during fluid handling device transfers. When the valved adapter of the present invention is in the normally closed position with no delivery device being mounted, the proximal end of the adapter presents a closed, substantially continuous surface to facilitate wiping. An added benefit of the instant valved adapter when used with a vascular access device, is that patient blood loss, in the event of an unintentional disconnect, is minimized because the valve is normally closed.

A valved adapter for a medical access device includes a body with a longitudinal axis. The body has a proximal first end with a proximal surface, a distal second end and a passageway therethrough that has an inside surface. The passageway has a chamber defined by a proximal shoulder and a segmented distal shoulder projecting inwardly from the surface of the passageway. The chamber is located intermediate the first end and second end of the body. The adapter includes a valve contained within the chamber for selectively obstructing and allowing fluid flow through the passageway. The valve includes an elongate resilient member with an axis substantially coaxial to the longitudinal axis of the body having a proximal end and a distal end. The elongate resilient member is axially compressed between the proximal shoulder and the distal shoulder, the compression biasing the valve to a normally closed position. The proximal end of the resilient member forms a substantially fluid tight seal with the proximal shoulder. The seal obstructs fluid flow through the passageway. The adapter further includes a pusher for selectively further axially compressing the resilient member so that the proximal end no longer forms a substantially fluid tight seal with the proximal shoulder thereby allowing fluid flow through the passageway.

The valved adapter preferably includes fittings at the distal end for mounting the adapter to medical access devices and for attachment of fluid delivery devices. These fittings may include threads, luer, snap-fit, bayonet and similar devices. The fitting on the delivery device being attached preferably includes provisions for engaging the pusher when the fluid delivery device is mounted in the proximal female luer fitting. In a preferred embodiment the valved adapter has a distal male luer fitting. The pusher extends proximally within the passageway forming a substantially continuous surface with the proximal surface of the first end and thereby closing the first end when the valve is in the normally closed position. When a fluid handling device having a male liner fitting is mounted on the proximal female luer fitting, the male fitting engages the pusher, opening the valve and allowing fluid access through the passageway.

The valved adapter for a medical device includes a cylindrical body with a longitudinal axis having a proximal first end with a female luer fitting that has a proximal surface. The body has a distal second end and a passageway therethrough with an inside surface. The passageway forms a chamber defined by a proximal shoulder and a segmented distal shoulder projecting inwardly from the surface of the passageway. The distal shoulder has openings between said segments. The chamber is located intermediate the first end and the second end. There is a valve contained within the chamber for selectively obstructing and allowing fluid flow through the passageway. The valve includes an elongate resilient member with an axis substantially coaxial to the longitudinal axis of the body. The resilient member has a distal end, a proximal end with a surface and preferably an outwardly projecting annulus intermediate the proximal end and the distal end. The resilient member is axially compressed between the proximal annular shoulder and the distal annular shoulder biasing the valve to a normally closed position with the intermediate annulus forming a fluid tight seal at the proximal shoulder. When the valve is closed, fluid flow through the passageway is obstructed. The resilient member extends proximally from the chamber so that the proximal surface of the resilient member is substantially continuous with the surface of the female luer fitting when said valve is in the normally closed position. The preferred resilient member includes a cavity having an opening in its proximal surface. A pusher with a proximal surface is positioned in the opening to the cavity so that the proximal surface of the pusher and the proximal surface of the resilient member are substantially continuous. The pusher selectively further axially compresses the resilient member so that the intermediate annulus of the elongate resilient member no longer forms a fluid tight seal with the proximal shoulder which opens the valve and allows fluid flow through the passageway. The pusher proximal surface, the resilient member proximal surface and the proximal surface of the female luer fitting form a substantially continuous surface closing said female luer fitting when said valve is in the normally closed position. The pusher is engaged opening the valve by mounting a fluid delivery device with a male liner fitting on the proximal female luer fitting which further axially compresses the resilient member and allows fluid flow through the passageway.

The valved adapter of the present invention may be incorporated into "Y" site adapters, manifolds, and other access devices. Additionally, the body may incorporate a fixed catheter tube or an additional sidearm for a tube. In any of these applications, any of the embodiments of the valved adapter may be incorporated or combinations of the embodiments may be incorporated into the same device.

### Brief Description of the Drawings

Fig 1 is a exploded cut-away perspective of an adapter of the present invention,
Fig. 2 is a cross sectional view of the adapter of Fig. 1 in the normally closed position;
Fig. 3 is a cross sectional view of the adapter of Fig. 1 including a proximally mounted fluid delivery device;
Fig. 4 is a top plan view of the adapter of Fig. 1;
Fig. 5 is a perspective view of the elongate resilient member shown folded or collapsed as it would be under further axial compression, i.e. the valve in the open position;
Fig. 6 is a cross sectional view of the elongate resilient member as shown in Fig. 5 along the line 6,6;
Fig. 7 is a cross sectional view of the elongate resilient member as shown in Fig. 5 along the line 7,7;
Fig. 8 is a cross sectional view of an embodiment of the present invention;
Fig. 9 is a top plan view of the embodiment of the present invention of Fig. 8;
Fig. 10 is an enlarged partial sectional detail of the embodiment of the present invention of Fig. 8;
Fig. 11 is a cross sectional view of the adapter of Fig. 8 including a proximally mounted fluid delivery device;
Fig. 12 shows a "y" connector device incorporating two valved adapters of the present invention mounted proximally on the "arms" of the "y";
Fig. 13 shows an embodiment of the valved adapter of Fig. 1 having a side arm for mounting a tube;
Fig. 14 shows a manifold device of the present invention with a plurality of the valved adapters of the present invention; and
Fig. 15 shows an embodiment of the invention of Fig. 1 including a fixed distal tube device.

### Detailed Description of the Invention

While this invention is satisfied by embodiments in many different forms, there is shown in the drawings and will herein be described, several embodiments of the invention with the understanding that the present disclosure is to be considered descriptive of the principles of the present invention and is not intended to limit the scope of the invention to the embodiments illustrated.

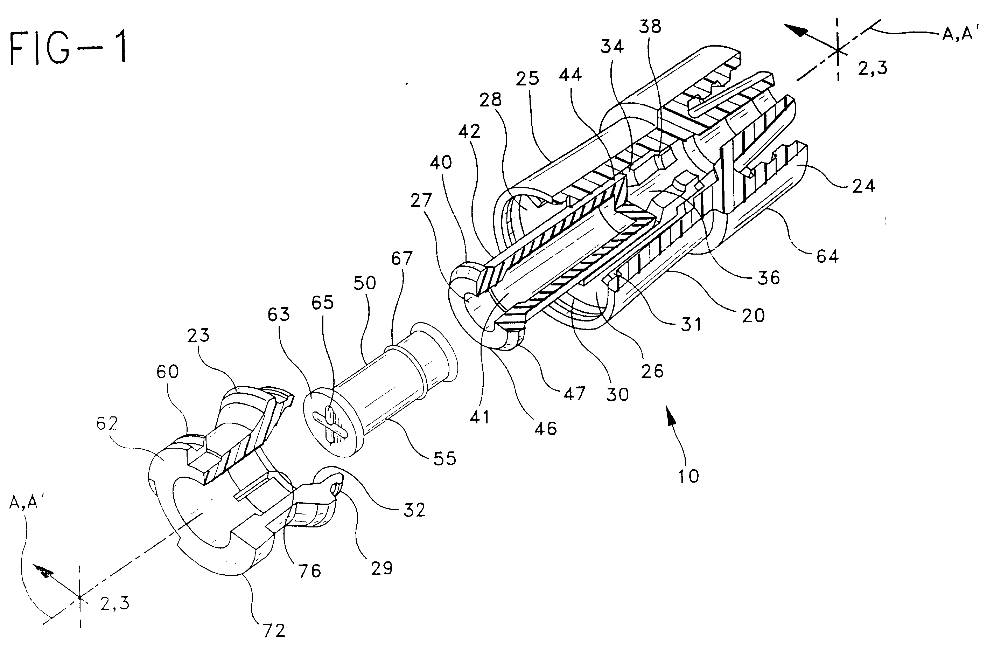

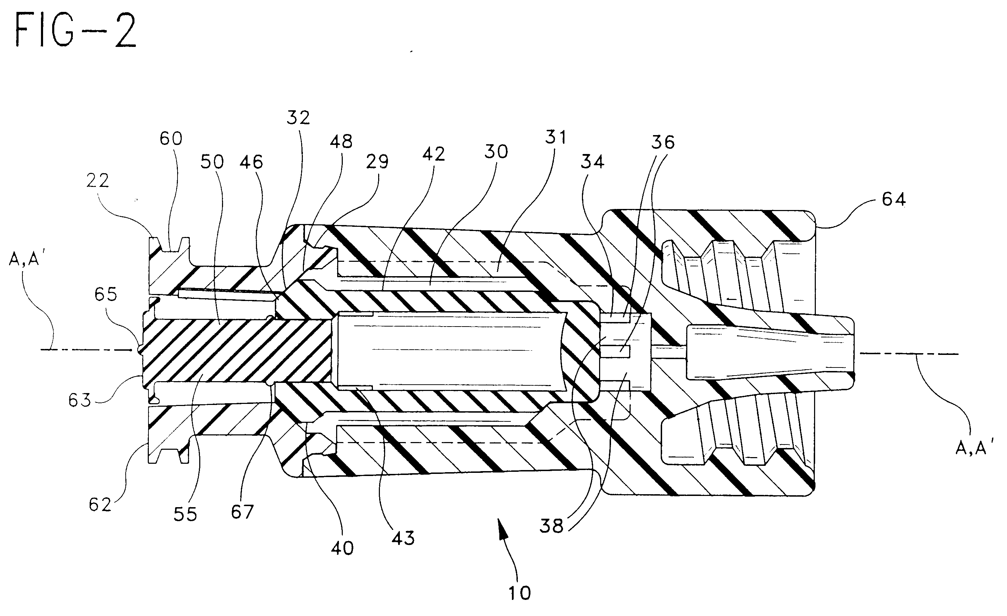

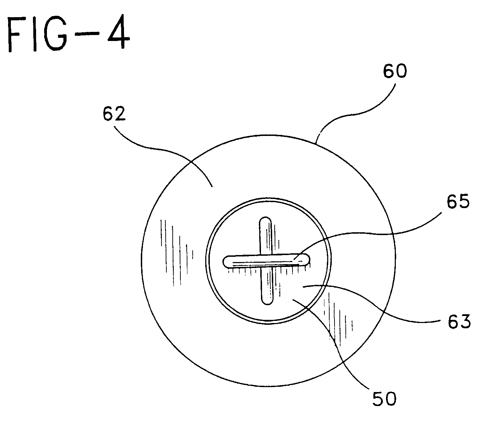

As shown to Figs. 1-4, a valved adapter 10 of the present invention for a medical access device includes a substantially cylindrical body 20 with a longitudinal axis A, a proximal first end 22 and a distal second end 24. Body 20 has a passageway 26 with an inside surface 28. Passageway 26 includes a chamber 30 having a plurality of axial channels 31. Chamber 30 is defined by a proximal shoulder 32 projecting inwardly from surface 28 and a segmented distal shoulder 34 projecting inwardly from surface 28 having openings 36 between segments 38. The adapter includes a valve mechanism 40 contained within chamber 30 for selectively obstructing and allowing fluid flow through passageway 26. Valve mechanism 40 includes an elongate resilient member 42 with an axis A' substantially coaxial to Axis A of body 20. Resilient member 42 has a distal end 44, a proximal end 46 and an internal cavity 41 therein with an opening 27 in proximal end 46. Resilient member 42 is axially compressed between proximal shoulder 32 and distal shoulder 34. The compression of resilient member 42 biases valve 40 to a normally closed position where proximal end 46 forms a fluid tight seal 48 with proximal shoulder 32 and obstructs fluid flow through passage 26. Adapter 10 further includes a pusher 50 for selectively further axially compressing resilient member 42 so that proximal end 46 no longer forms fluid tight seal 48 with proximal shoulder 32, thereby opening valve 40 and allowing fluid flow through the passageway. Pusher 50 is fit within opening 27 into cavity 41. Preferably chamber 30 includes plurality of axial channels 31 to facilitate fluid flow around elongate resilient member 42 when valve 40 is open.

Proximal end 22 and distal end 24 of adapter 10 include fittings 60 and 64 respectively to facilitate attachment of fluid delivery and access devices. The fitting 60 at proximal end 24 includes a female luer fitting and fitting 64 at distal end 24 includes a male liner fitting as shown in the drawings. Fittings 64 may also include other types of fitting for particular applications. The other types of fittings may include, but are not limited to threads, snap-fit, bayonet and the like.

Preferably fluid tight seal 48 includes a chamfered surface 47 on proximal end 46 of resilient member 42 that engages a conjugate frusto-conical surface 49 located on proximal shoulder 32 that is coaxial to longitudinal axis A of the body.

Pusher 50 has a proximally extending portion 55 extending proximally from cavity 41 within proximal end 22 so that attachment of fluid delivery device 70 will engage pusher 50 thereby farther axially compressing resilient member 42, opening valve 40 so that fluid may flow through passageway 26. Proximal end 22 is a female liner fitting 60 having a proximal surface 62. Proximal extension 55 has a proximal surface 63 which is substantially continuous with surface 62 of female luer fitting 60, closing fitting when valve 40 is in the normally closed position, so that the proximal surfaces 62 and 63 may be wiped by an operator when no delivery device is mounted on fitting 60.

Preferably, pusher proximal surface 63 preferably has elements 65 projecting proximally from surface 63 to facilitate fluid flow from fluid delivery device 70 which preferably has a male luer fitting 72 for attachment to proximal end 22 of the adapter. Elements 65 also serve to prevent adherence of male luer fitting to surface 63 of the pusher. Pusher 50 farther includes ring 67 to hold pusher 50 in resilient member 42 at opening 27. A proximal portion 74 of passageway 26 preferably includes a plurality of flow channels 76 to facilitate fluid flow from delivery device 70 around the pusher proximal extension into the adapter. Portion 55 extends into proximal female luer fitting 60 so that when the fluid delivery device fitting 72 is a male luer mounted on female luer 60 at the proximal end, male luer 72 will engage pusher 50. The engagement of pusher 50 further axially compresses resilient member 42 and opens valve 40 so that fluid may flow through the passageway.

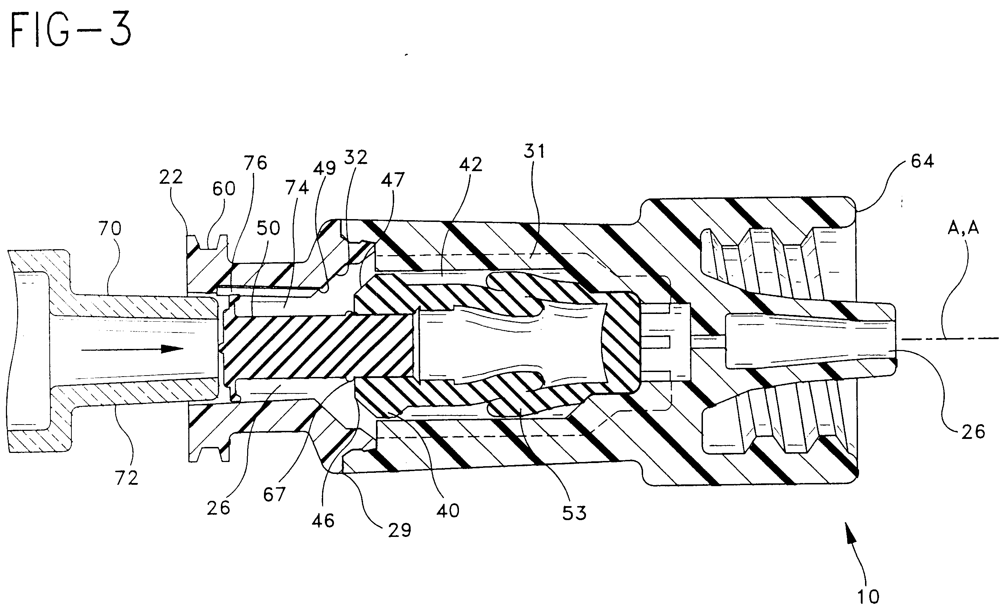

In Fig. 3, the cross-sectional view of the adapter 10 shows fluid delivery device 70 with male liner 72 mounted on female liner 60 and contacting pusher 50 so that the pusher axially farther compresses resilient member 42 thus separating chamfer surface 47 from frusto-conical surface 49 opening valve 40 and allowing fluid flow from delivery device 70 through the passageway.

Referring to Figs. 1-3, a method for assembling a valved adapter 10 includes providing body portion 20 having longitudinal axis A, proximal first end 22 and distal second end 24 with passageway 26 therethrough having inside surface 28. The passageway has chamber 30 defined by proximal shoulder 32 and segmented distal shoulder 34 projecting inwardly from surface 28. Distal shoulder 34 has openings 36 therethrough between segments 38. Chamber 30 is intermediate first end 22 and second end 24. Body 20 preferably includes two parts, a proximal body part 23 and a distal body part 25, with a parting line 29 transverse axis A located intermediate proximal shoulder 32 and distal shoulder 34.

The method includes providing valve 40 for containment within chamber 30 for selectively obstructing and allowing fluid flow through passageway 26. The valve includes elongate resilient member 42. The method further includes providing pusher 50 for selectively further compressing elongate resilient member 42 to allow fluid flow.

The method of assembly includes placing elongate resilient member distal end 44 in distal body part 25 so that distal end 44 contacts distal shoulder 34 and the parts are coaxially aligned. Pusher 50 is then axially placed in cavity 41 through opening 27 of elongate resilient member 42, so that pusher proximal extension 55 extends proximally. Proximal body portion 23 is then placed in coaxial alignment with distal body part 25 and placed over pusher proximal extension 55 so that pusher proximal surface 63 is substantially continuous with surface 62 of proximal female luer fitting 60. Proximal body part 23 is then axially advanced to distal body part 25 so that frustoconical surface 49 on proximal shoulder 32 contacts chamfer surface 47 axially compressing resilient elongate member 42 biasing valve 40 in the normally closed position. The assembly is completed by fixedly attaching proximal part 23 to distal part 25. Methods of bonding molded parts known in the art such as adhesive, solvent, press-fitting, spin welding, thermal welding, RF welding, ultra-sonic welding and the like may be used.

In a preferred embodiment where body 20 is formed from polycarbonate, solvent bonding is preferred, but other methods known in the art of plastic bonding are satisfactory.

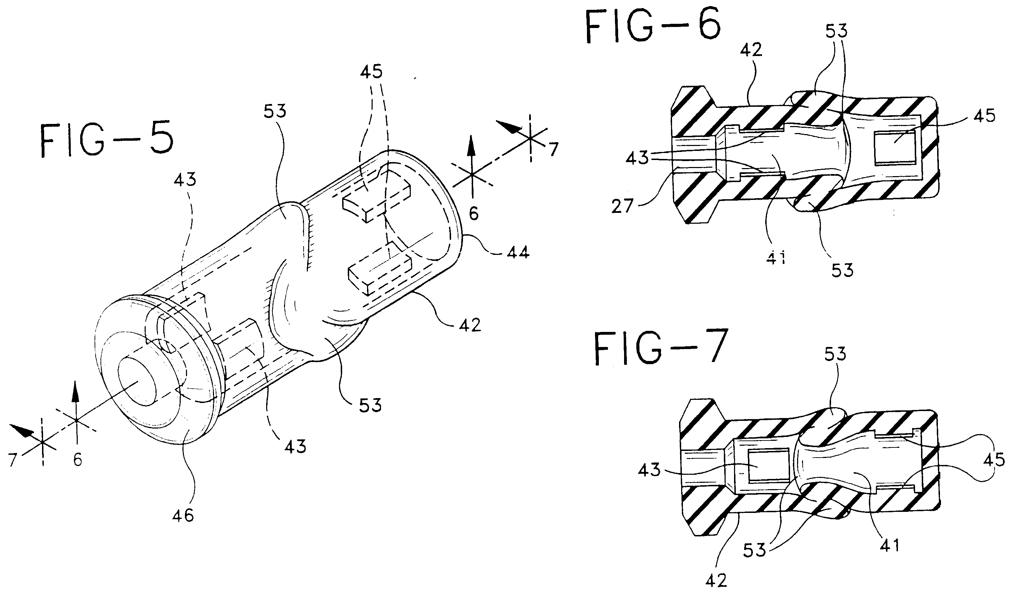

Since, according to industry standards, a fluid delivery device male luer fitting may vary in engagement length as much as 2.5mm and still be considered within the standard, it is important that a valved adapter, noting that the fitting at proximal end 22 is a female luer fitting, be able to accommodate all acceptable variants and still function. Figs. 5, 6 and 7 display elongate resilient member 42 in the further axially compressed state, i.e., where proximal end 46 no longer forms the fluid tight seal with the proximal shoulder. Resilient member 42 has internal cavity 41 having a plurality of proximal internal projections 43 to retain pusher 50 within the proximal portion of the resilient member. Cavity 41 includes a plurality of distal internal ribs 45 which, along with projections 43, serve to direct the collapse of the resilient member under further axial compression to a region of collapse 53 which allows pusher 50 a greater range of travel than would be possible if only axial compression were utilized. Preferably, there are two diametrically opposed proximal internal projections and two diametrically opposed distal ribs transverse the longitudinal axis. Preferably the diametric lines of the proximal internal projections and distal ribs are orthogonal. The ability of resilient member 42 to collapse in directed region 53 is of particular importance, because the entire length of a male liner on a delivery device must be accommodated in order for the fluid handling device to be fully mounted on proximal female liner fitting 60a. Resilient member 42 of the present invention with the directed collapse provided by cavity 41, projections 43 and ribs 45 allows male liner fitting 72 at the "long" (i.e., "thin") end of the allowable dimension to be fully mounted and also allows for a male liner 72 at the "short" (i.e., "fat") end of the dimension to open the valve. Figs. 6 and 7 further demonstrate how the resilient member collapses showing the directed region of collapse 53 and display the preferred locations of the proximal projections and the distal ribs

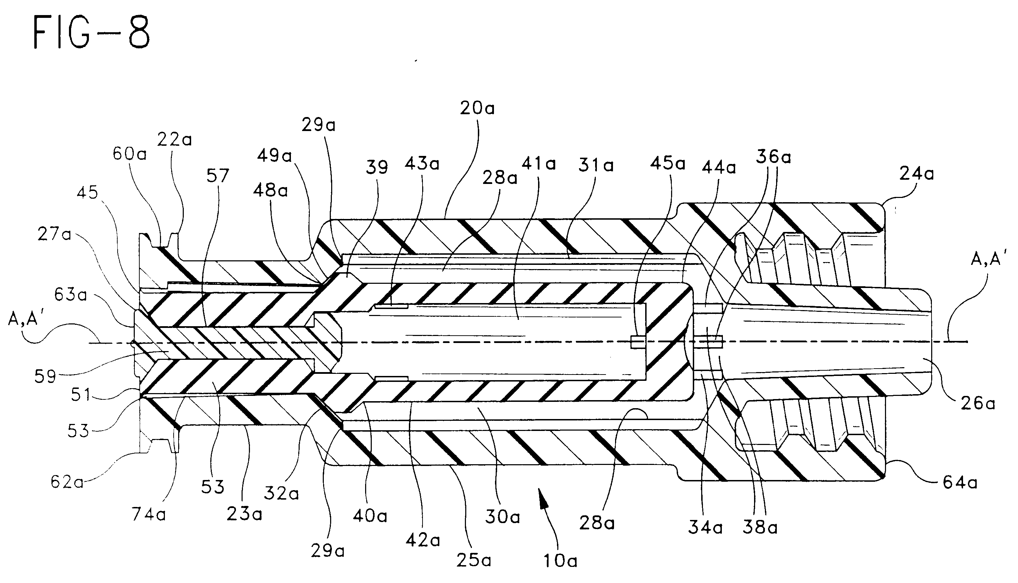

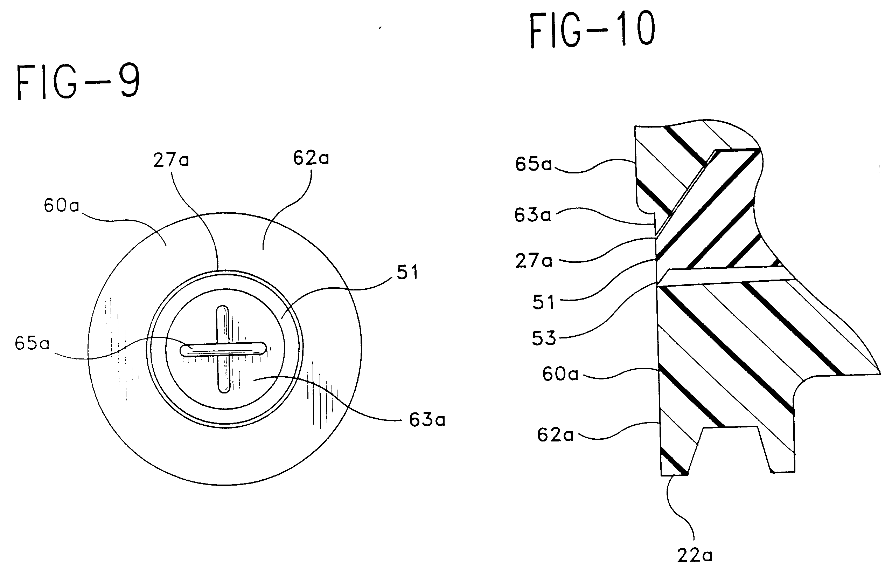

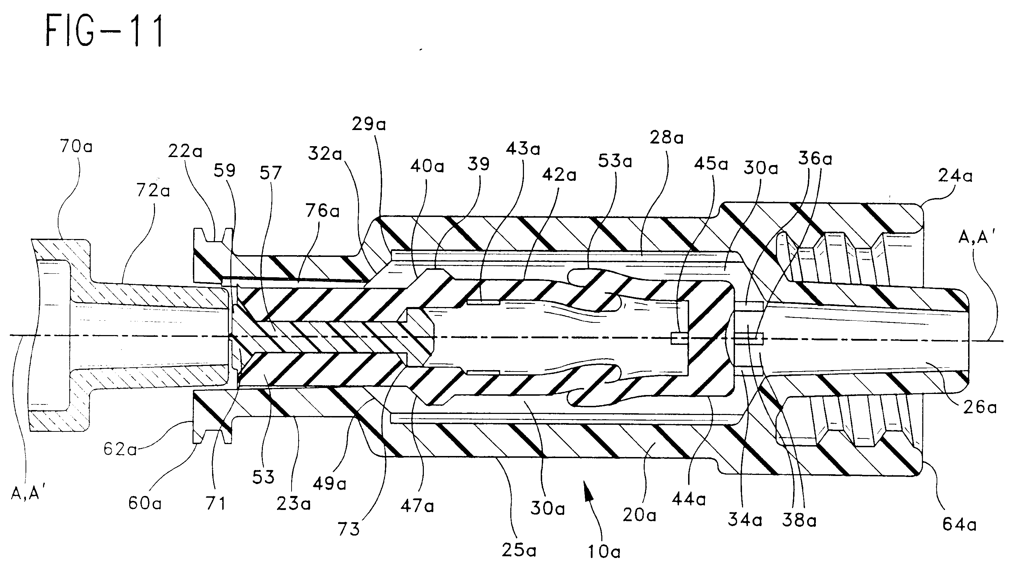

A preferred alternate embodiment to the embodiment shown in Figs. 1-4 of the valved adapter of the present invention is shown in the Figs. 8-11. In this preferred embodiment, there are elements similar in structure and function to the valved adapter of the present invention as shown in Figs. 1-7. Accordingly, substantially similar components that perform substantially similar functions will be numbered identically to those components of the embodiment except that the suffix a will be used to identify those components in Figs. 8-11. As shown to Figs. 8-11, a valved adapter 10a of the present invention for a medical access device includes a substantially cylindrical body 20a with a longitudinal axis A, a proximal first end 22a and a distal second end 24a. Body 20a has a passageway 26a with an inside surface 28a. Passageway 26a includes a chamber 30a having a plurality of axial channels 31a. Chamber 30a is defined by a proximal shoulder 32a projecting inwardly from surface 28a and a segmented distal shoulder 34a projecting inwardly from surface 28a having openings 36a between segments 38a. The adapter includes a valve mechanism 40a contained within chamber 30a for selectively obstructing and allowing fluid flow through passageway 26a. Valve mechanism 40a includes an elongate resilient member 42a with an axis A' substantially coaxial to Axis A of body 20a. Resilient member 42a has a distal end 44a, a proximal end 45 having a surface 51 with a flexible extension 53; an outwardly projecting annulus 39 substantially transverse said axis located intermediate said proximal end and said distal end and an internal cavity 41a having an opening 27a in proximal end 45. Resilient member 42a farther includes a plurality of proximal internal projections 43a and distal ribs 45a. Preferably there are two diametrically opposed internal projections 43a located within cavity 41a internal to annulus 39 and two diametrically opposed distal ribs 45a, preferably the diametric opposition lines for projections 43a and ribs 45a are transverse longitudinal axis A and orthogonal to each other. Resilient member 42a is axially compressed between proximal shoulder 32a and distal shoulder 34a. The compression of resilient member 42a biases valve 40a to a normally closed position where intermediate annulus 39 forms a fluid tight seal 48a with proximal shoulder 32a and obstructs fluid flow through passage 26a. Adapter 10a further includes a pusher 57 for selectively farther axially compressing resilient member 42a so that intermediate annulus 39 no longer forms fluid tight seal 48a with proximal shoulder 32a, thereby opening valve 40a and allowing fluid flow through the passageway. Preferably chamber 30a includes plurality of axial channels 31a to facilitate fluid flow around elongate resilient member 42a when valve 40a is open. As is shown in Fig. 11, resilient member 42a collapses at region of collapse 53a directed by internal projections 43a and the distal ribs 45a upon farther axial compression in the same manner as shown in the Figs. 5-7 for the first embodiment.

Proximal end 22a and distal end 24a of adapter 10a include fittings 60a and 64a respectively. The presence of these fittings facilitates attachment of fluid delivery and access devices. Fitting 60a at proximal end 22a includes a female liner fitting and fitting 64a at distal end 24a preferably includes a male luer fitting as shown in the drawings. Fittings 64a may also include other types of fitting for particular applications. The other types of fittings may include, but are not limited to threads, snap-fit, bayonet and the like.

Preferably fluid tight seal 48a includes a chamfered surface 47a on intermediate annulus 39 of resilient member 42a that engages a conjugate frusto-conical surface 49a located on proximal shoulder 32a that is coaxial to longitudinal axis A of the body.

Pusher 57 has a proximally extending portion 59 extending proximally within proximal end 22a so that attachment of fluid delivery device 70a will engage pusher 57 thereby farther axially compressing resilient member 42a, opening valve 40a so that fluid may flow through passageway 26a. Proximal end 22a is a female liner fitting 60a having a proximal surface 62a. Proximal extension 59 has a proximal surface 63a which is substantially continuous with surface 62a of female luer fitting 60a and surface 51 of extension 53 of the resilient member, closing fitting 60a when valve 40a is in the normally closed position, so that the proximal surfaces 51, 62a and 63a may be wiped by an operator when no delivery device is mounted on fitting 60a.

Preferably, pusher proximal surface 63a preferably has elements 65a projecting proximally from surface 63a to facilitate fluid flow from fluid delivery device 70a which preferably has a male liner fitting 72a for attachment to proximal end 22a of the adapter. Elements 65a also serve to prevent adherence of male liner fitting to surface 63a of the pusher. Pusher 57 further includes elements 71 and 73 to retain pusher 57 in resilient member 42a. A proximal portion 74a of passageway 26a preferably includes a plurality of flow channels 76a to facilitate fluid flow from delivery device 70a around the pusher proximal extension into the adapter.

In Fig. 11, the cross-sectional view of the adapter 10a shows fluid delivery device 70a with male liner 72a mounted on female liner 60a and contacting portion 59 of pusher 57 so that the pusher axially farther compresses resilient member 42a thus separating chamfer surface 47a from frusto-conical surface 49a opening valve 40a and allowing fluid flow from delivery device 70a through the passageway.

Referring to Figs. 8-11, a method for assembling a valved adapter 10a includes providing body portion 20a having longitudinal axis A, proximal first end 22a and distal second end 24a with passageway 26a therethrough having inside surface 28a. The passageway has chamber 30a defined by proximal shoulder 32a and segmented distal shoulder 34a projecting inwardly from surface 28a. Distal shoulder 34a has openings 36a therethrough between segments 38a. Chamber 30a is intermediate first end 22a and 24a. Body 20a preferably includes two parts, a proximal body part 23a and a distal body part 25a, with a parting line 29a transverse axis A located intermediate proximal shoulder 32a and distal shoulder 34a.

The method includes providing valve 40a for containment within chamber 30a for selectively obstructing and allowing fluid flow through passageway 26a. The valve includes elongate resilient member 42a. The method further includes providing pusher 57 for selectively farther compressing elongate resilient member 42a to allow fluid flow.

The method of assembly includes placing elongate resilient member distal end 44a in distal body part 25a so that distal end 44a contacts distal shoulder 34a and the parts are coaxially aligned. Pusher 57 is then axially placed in opening 27a within proximal end 45 of elongate resilient member 42a, so that pusher proximal extension 59 extends proximally. Proximal body portion 23a is then placed in coaxial alignment with distal body part 25a and placed over pusher proximally extending portion 59 so that pusher proximal surface 63a is substantially continuous with surface 62a of proximal female luer fitting 60a and resilient member proximal surface 51. Proximal body part 23a is then axially advanced to distal body part 25a so that frusto-conical surface 49a on proximal shoulder 32a contacts the chamfer surface 47a on annulus 39 axially compressing resilient elongate member 42a and biasing valve 40a to the normally closed position. The assembly is completed by fixedly attaching proximal part 23a to distal part 25a. Methods of bonding molded parts known in the art such as adhesive, solvent, press-fitting, thermal welding, RF welding, ultra-sonic welding and the like may be used. In a preferred embodiment where body 20a is formed from polycarbonate, solvent bonding is preferred, but other methods known in the art of plastic bonding are satisfactory. When other methods of bonding are used, the order of the placement steps may be changed, for example, when proximal body portion 23a and distal body part 25a are bonded by spin weld or ultra-sonic techniques, resilient member 42a may be inserted into chamber 30a after the body portions are bonded.

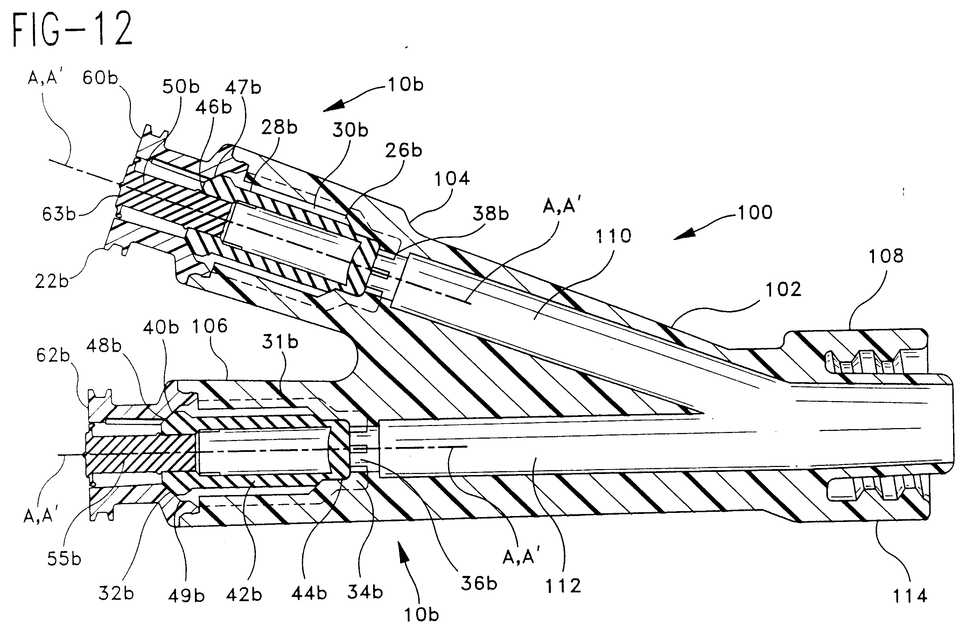

Applications incorporating an embodiment of the valved adapter of the present invention are shown in Figs. 12.-15. In the Figs. 12-15, the applications illustrate the embodiment as shown in Figs. 1-7, but one skilled in the art of medical access devices will recognize that the applications could equally incorporate the preferred embodiment of Figs. 8-11, or, in the cases where more than one valved adapter is incorporated into the device, both types could be used in combination. For these applications, elements having similar function to those elements in Figs 1-7 will have similar reference characters with the exception that the suffixes b-e will be used in Figs. 12-15 respectively.

As shown in Fig. 12, a valved "Y" adapter 100 of the present invention for a medical access device includes body portion 102 having a first proximal branch 104 and a second proximal branch 106 joined at a distal end 108. Proximal branches 104 and 106 each have passageways 110 and 112 respectively therethrough commonly joined at distal end 108. Each proximal branch is proximally terminated by a valved adapter 10b of the present invention having a longitudinal axis A. Valved adapters 10b have proximal first ends 22b and distal second ends 24b joined to body portion 100. Each adapter 10b has a passageway 26b with an inside surface 28b fluidly communicative with passageways 110 and 112 respectively. Passageways 26b include chambers 30b having a plurality of axial channels 31b. Chambers 30b are defined by proximal shoulders 32b projecting inwardly from surfaces 28b and segmented distal shoulders 34b projecting inwardly from surfaces 28b having openings 36b between segments 38b. The adapters include valve mechanisms 40b contained within chambers 30b for selectively obstructing and allowing fluid flow through passageways 26b. Valve mechanisms 40b include elongate resilient members 42b with axis A' substantially coaxial to Axis A of valved adapter 10b. Resilient members 42b have distal ends 44b and a proximal ends 46b. Resilient members 42b are axially compressed between proximal shoulders 32b and distal shoulders 34b. The compression of resilient members 42b biases valves 40b to normally closed positions where proximal ends 46b form fluid tight seals 48b with proximal shoulders 32b and obstructs fluid flow through passages 26b. Valved adapters 10b farther include pushers 50b for selectively further axially compressing resilient members 42b so that proximal ends 46b no longer form fluid tight seals 48b with proximal shoulder 32b, thereby individually opening valves 40b and allowing fluid flow through each of the passageways. Preferably chambers 30b include pluralities of axial channels 31b to facilitate fluid flow around elongate resilient members 42b when each valve 40b is open.

Proximal ends 22b valved adapters 10b include fittings 60b to facilitate attachment of fluid delivery and access devices. Fittings 60b at proximal ends 24b include female luer fittings, with a male luer fitting 114 preferred at common distal end 108. Fittings 114 may also include other types of fitting for particular applications. The other types of fittings may include, but are not limited to threads, snap-fit, bayonet and the like.

Preferably fluid tight seals 48b include chamfered surfaces 47b on proximal ends 46b of resilient members 42b that engage conjugate frusto-conical surfaces 49b located on proximal shoulders 32b that are coaxial to longitudinal axis A of the valved adapter.

Pushers 50b have proximally extending portions 55b extending proximally within proximal ends 22b so that attachment of fluid delivery devices will engage pushers 50b thereby further axially compressing resilient members 42b, individually opening valves 40b so that fluid may flow through passageways 26b. Proximal ends 22b are female luer fittings 60b having proximal surfaces 62b. Proximal extensions 55b have proximal surfaces 63b which are substantially continuous with surfaces 62b of female liner fittings 60b when valves 40b are in the normally closed position, so that the proximal surfaces 62b and 63b may be wiped by an operator

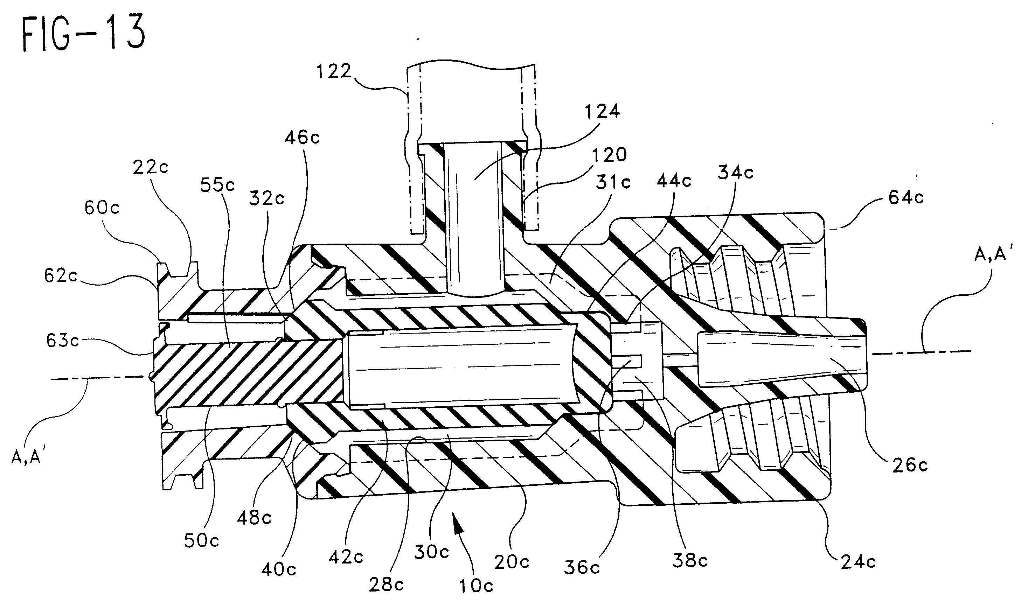

As shown in Fig. 13, a valved adapter 10c of the present invention for a medical access device includes a substantially cylindrical body 20c with a longitudinal axis A, a proximal first end 22c and a distal second end 24c. Body 20c has a passageway 26c with an inside surface 28c and a sidearm 120 for mounting a tubing 122. Passageway 26c includes a chamber 30c having a plurality of axial channels 31c. Sidearm 120 has a passageway 124 therethrough fluidly communicative with passageway 26c at chamber 30c. Chamber 30c is defined by a proximal shoulder 32c projecting inwardly from surface 28c and a segmented distal shoulder 34c projecting inwardly from surface 28c having openings 36c between segments 38c. The adapter includes a valve mechanism 40c contained within chamber 30c for selectively obstructing and allowing fluid flow through passageway 26c. Valve mechanism 40c includes an elongate resilient member 42c with an axis A' substantially coaxial to Axis A of body 20c. Resilient member 42 has a distal end 44c and a proximal end 46c. Resilient member 42c is axially compressed between proximal shoulder 32c and distal shoulder 34c. The compression of resilient member 42c biases valve 40c to a normally closed position where proximal end 46c forms a fluid tight seal 48c with proximal shoulder 32c and obstructs fluid flow through passage 26c. Adapter 10c further includes a pusher 50c for selectively further axially compressing resilient member 42c so that proximal end 46c no longer forms fluid tight seal 48c with proximal shoulder 32c, thereby opening valve 40c and allowing fluid flow through the passageway. Preferably chamber 30c includes plurality of axial channels 31c to facilitate fluid flow around elongate resilient member 42c when valve 40c is open.

Proximal end 22c and distal end 24c of adapter 10c include fittings 60c and 64c respectively to facilitate attachment of fluid delivery and access devices. Fitting 60c at proximal end 24c includes a female luer fitting and fitting 64c at distal end 24c preferably includes a male luer fitting as shown in Fig. 13. Fittings 64c may also include other types of fitting for particular applications. The other types of fittings may include, but are not limited to threads, snap-fit, bayonet and the like. Preferably fluid tight seal 48c includes a chamfered surface 47c on proximal end 46c.

Pusher 50c has a proximally extending portion 55c extending proximally within proximal end 22c so that attachment of fluid delivery device will engage pusher 50c thereby farther axially compressing resilient member 42c, opening valve 40c so that fluid may flow through passageway 26c. Proximal end 22c is a female luer fitting 60c having a proximal surface 62c. Proximal extension 55c has a proximal surface 63c which is substantially continuous with surface 62c of female luer fitting 60c when valve 40c is in the normally closed position, so that the proximal surfaces 62c and 63c may be wiped by an operator

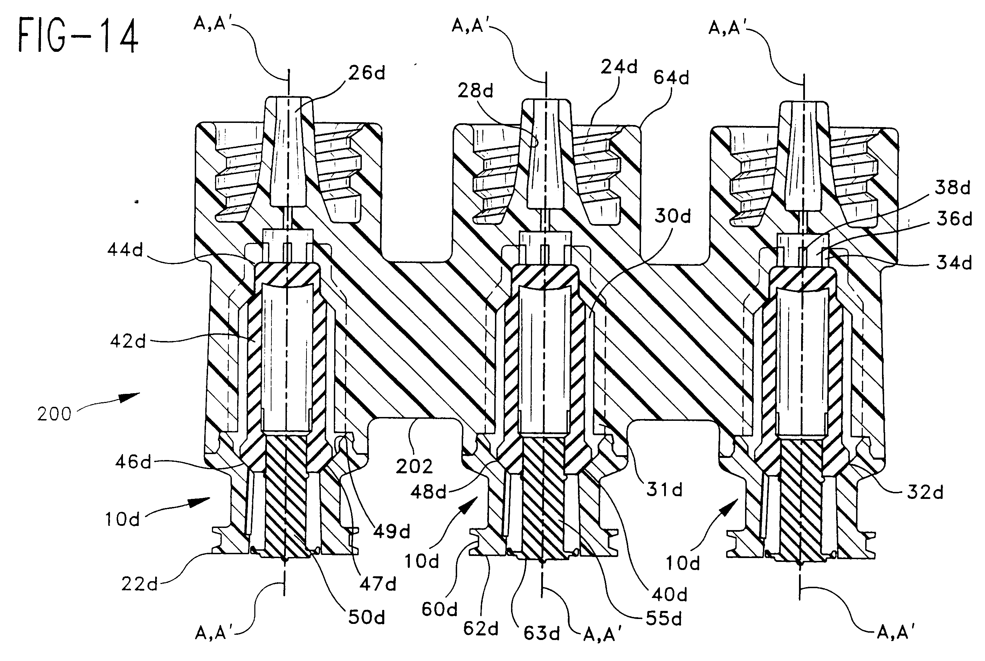

As shown in Fig. 14 a manifold adapter 200 includes a body portion 202 for mounting a plurality of valved adapters 10d of the present invention, each with a longitudinal axis A, a proximal first end 22d and a distal second end 24d. Each valved adapter 10d includes a passageway 26d with an inside surface 28d. Passageways 26d include chambers 30d having a plurality of axial channels 31d. Chambers 30d are defined by proximal shoulders 32d projecting inwardly from surfaces 28d and segmented distal shoulders 34d projecting inwardly from surfaces 28d having openings 36d between segments 38d. Each adapter includes a valve mechanism 40d contained within chamber 30d for selectively obstructing and allowing fluid flow through individual passageways 26d. Valve mechanisms 40d include elongate resilient member 42d with axis A' substantially coaxial to Axis A of valved adapter 10d. Resilient members 42d have distal ends 44d and proximal ends 46d. Resilient members 42d are axially compressed between proximal shoulders 32d and distal shoulders 34d. The compression of resilient members 42d biases valves 40d to normally closed positions where proximal ends 46d form fluid tight seals 48d with proximal shoulders 32d and obstructs fluid flow through passages 26d. Each valved adapter 10d farther includes a pusher 50d for selectively further axially compressing resilient members 42d so that proximal ends 46d no longer forms fluid tight seals 48d with proximal shoulders 32d, thereby opening valves 40d and allowing fluid flow through each individual passageway 26d. Preferably chambers 30d include pluralities of axial channels 31d to facilitate fluid flow around elongate resilient members 42d when valves 40d are open.

Proximal ends 22d are female liner fittings 60d having proximal surfaces 62d. Preferably proximal extensions 55d have proximal surfaces 63d which are substantially continuous with surfaces 62d of female luer fittings 60d when valves 40d are in the normally closed position, so that the proximal surfaces 62d and 63d may be wiped by an operator. Manifold 200 includes female luer fittings at proximal ends 22d and preferably male luer fittings 64d at distal ends 24a as shown in Fig. 14. Fittings 64d may also include other types of fitting for particular applications. The other types of fittings may include, but are not limited to threads, snap-fit, bayonet and the like.

Preferably fluid tight seals 48d include chamfered surfaces 47d on proximal ends 46d of resilient members 42d that engage conjugate frusto-conical surfaces 49d located on proximal shoulders 32d that are coaxial to longitudinal axis A of the valved adapters.

Pushers 50d have proximally extending portions 55d extending proximally within proximal ends 22d so that attachment of fluid delivery devices will engage pushers 50d thereby further individually axially compressing resilient members 42d, individually opening valves 40d so that fluid may flow through passageways 26d. Proximal ends 22d have female liner fittings 60d having proximal surfaces 62d.

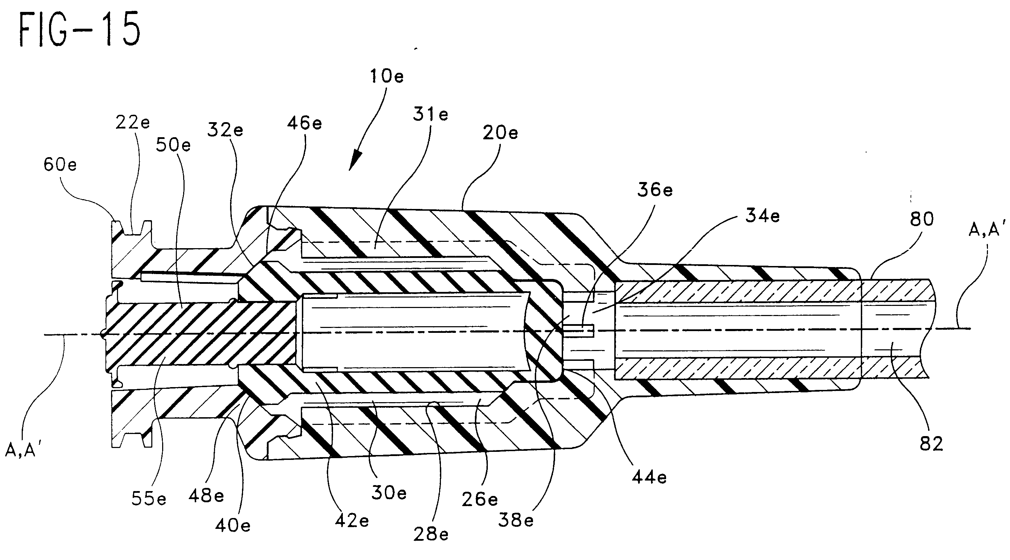

As shown in Fig. 15, a valved adapter 10e of the present invention for a medical access device includes a substantially cylindrical body 20e with a longitudinal axis A, a proximal first end 22e and a distal second end 24e. Body 20e has a passageway 26e with an inside surface 28e. Passageway 26e includes a chamber 30e with an inside surface 28e. Passageway 26e includes a chamber 30e having a plurality of axial channels 31e. Chamber 30e is defined by a proximal shoulder 32e projecting inwardly from surface 28e and a segmented distal shoulder 34e projecting inwardly from surface 28e having openings 36e between segments 38e. The adapter includes a valve mechanism 40e contained within chamber 30a for selectively obstructing and allowing fluid flow through passageway 26a. Valve mechanism 40e includes an elongate resilient member 42e with an axis A' substantially coaxial to Axis A of body 20e. Resilient member 42e has a distal end 44e and a proximal end 46e. Resilient member 42e is axially compressed between proximal shoulder 32e and distal shoulder 34e. The compression of resilient member 42e biases valve 40e to a normally closed position where proximal end 46e forms a fluid tight seal 46e with proximal shoulder 32e and obstructs fluid flow through passage 26e. Adapter 10e further includes a pusher 50e for selectively further axially compressing resilient member 42e so that proximal end 46e no longer forms fluid tight seal 48e with proximal shoulder 32e, thereby opening valve 40e and allowing fluid flow through the passageway. Preferably chamber 30e includes plurality of axial channels 31e to facilitate flow through the passageway. Preferably chamber 30e includes plurality of axial channels 31e to facilitate fluid flow around elongate resilient member 42e when valve 40e is open.

Proximal end 22e of adapter 10e includes fitting 60e to facilitate attachment of fluid delivery and access devices. Fitting 60e at proximal end 22e includes a female luer fitting and distal end 24e preferably includes a tubing 80 with a passageway 82 therethrough which is fluidly communicative with passageway 26e. Tubing 80 may be a catheter suitable for vascular implantation, a catheter for implantation into a body cavity, a feeding tube or other tubing for medical applications. The only requirement would be that a delivery device have a fitting suitable for engaging pusher 50e when the delivery device is mounted. Other suitable types of flttings may include, but are not limited to threads, snap-fit, bayonet and the like.

Preferably fluid tight seal 48e includes a chamfered surface 47e on proximal 46e of resilient member 42e that engages a conjugate frusto-conical surface 49e located on proximal shoulder 32e that is coaxial to longitudinal axis A of the body.

Pusher 50e has a proximally extending portion 55e extending proximally proximal end 22e so that attachment of the fluid delivery device will engage the pusher thereby farther axially compressing resilient member 42e, opening valve 40e so that fluid may flow through passageway 26e into tubing 80. Proximal end 22e is a female liner fitting having a surface which is substantially continuous with the surface of the pusher.

Components for the body portion of the valved adapter may be formed from plastic resins such as polystyrene, polycarbonate, polypropylene, polyacetal, polyacrylate, polysulfone, polyamide, polyvinylchloride, alloys thereof and others which have the required properties of dimensional stability and strength. The preferred resin is polycarbonate that is preferably injection molded to form proximal and distal body portions of the individual valved adapter and also body portions of the "Y" type and manifold embodiments. The elongate resilient members of the several embodiments of the present invention may be formed from natural rubber, thermoplastic elastomer, chloroprene, silicone rubber, ethylene-propylene-diene monomer (EPDM) or other elastomers having low compression set tendencies and suitable biocompatability. Preferably, the elongate resilient member is formed by compression molding from silicone rubber having a durometer between about 45 and about 70 Shore A.

## Claims

1. A valved adapter (10) for a medical device comprising:
a body (20) with a longitudinal axis (A) having a proximal first end (22) comprising a female liner fitting (60) having a proximal surface, a distal second end (24) and a passageway (26) therethrough having an inside surface (28), said passageway (26) having a chamber (30) defined by a proximal shoulder (32) and a distal shoulder (34) projecting inwardly from said surface (28) of said passageway (26), said chamber (30) being intermediate said first end (22) and said second end (24);
a valve (40) contained within said chamber (30) for selectively obstructing and allowing fluid flow through said passageway (26), said valve (40) comprising an elongate resilient member (42) having an axis (A') substantially coaxial to said longitudinal axis (A) of said body (20) and having a distal end (44) and a proximal end (46), said resilient member (42) being axially compressed between said proximal shoulder (32) and said distal shoulder (34), said compression of said elongate resilient member (42) alone biasing said valve (40) to a normally closed position thereby obstructing fluid flow through said passageway (26); and
a pusher (50) on said elongate resilient member (42) for selectively further axially compressing said elongate resilient member (42) thereby opening said valve (40) and allowing fluid flow through said passageway (26), said pusher (50) having a proximally extending portion (55) within said female liner fitting (60) so that when a fluid handling device having a male liner fitting is mounted on said proximal end (22) of said adapter (10) having said female liner fitting (60), the male liner fitting will engage said pusher (50) thereby further compressing said resilient member (42) so that fluid may flow through said passageway (26), said pusher proximally extending portion (55) having a surface (63) forming a substantially continuous surface with said female luer fitting (60) proximal surface thereby closing said female luer fitting (60) when said valve (40) is in said normally closed position;
characterised in that the elongate resilient member (42) is formed from an elastomer, the distal shoulder (34) is a segmented distal shoulder (34) having openings (36) therethrough between said segments (38),
said proximal end of said resilient member (42) forms a fluid tight seal (48) with said proximal shoulder (32) upon said compression of said elongate resilient member (42) biasing said valve (40) to a normally closed position thereby obstructing fluid flow through said passageway (26), and
said pusher (50) is for selectively further axially compressing said elongate resilient member (42) so that said proximal end (46) of said elongate resilient member (42) no longer forms said fluid tight seal with said proximal shoulder (32).

2. The adapter (10) of claim 1 wherein said surface of said proximally extending portion (55) of said pusher (50) further includes a proximal projection (65) to facilitate fluid flow from the male luer fitting into said adapter (10) from the male luer fitting.

3. The adapter (10) of claim 1 wherein said distal end (24) of said body (20) comprises attachment means for attachment of said adapter (10) to a medical device.

4. The adapter (10) of claim 3 wherein said distal end attachment means of said body (20) comprises a male luer fitting.

5. The adapter (10) of claim 3 wherein said distal end of said body (20) comprises a fixedly attached catheter tube.

6. The adapter (10) of claim 1 wherein said chamber (30) includes a plurality of channels (31) for facilitating fluid flow around said elongate resilient member (42) when said valve (40) is open.

7. The adapter (10) of claim 1 wherein said elongate resilient member (42) further includes a cavity (41) substantially coaxial to said axis (A) of said member (42), said cavity (41) having an open end (27) at said proximal end (46) of said member (42) for receiving said pusher (50), said cavity (41) having a plurality of inward projections (43) and ribs (45), said plurality comprising proximal projections and distal ribs within said cavity (41), said proximal projections (43) serving to retain said pusher (50) and, in conjunction with said distal ribs (45), serving to form a region (53) of collapse when said resilient member (42) is further axially compressed for opening said valve (40).

8. The adapter (10) of claim 1 wherein said plurality of projections (43) and ribs (45) comprises two diametrically opposed proximal projections and two diametrically opposed distal ribs, said proximal projections and said distal ribs being arrayed with said diametric opposition lines being orthogonal.

9. The adapter (10c) of claim 1 wherein said body (20c) is cylindrical and further includes a side arm (120) having a passageway (26c) therethrough fluidly connected to said chamber (30c), said side arm (120) for mounting a tube for fluid access to said chamber (30c).

10. A "Y" type adapter (100) having two proximal arms (104, 106) each comprising valved adapters (10b) of claim 1 with said distal second ends (108) of said valved adapter (10) being fluidly connected and joined to a common male liner fitting (114).

11. A manifold connector (200) comprising a plurality of valved adapters (10d) of claim 1 being fixedly attached together.

## Patentansprüche

1. Mit Ventil versehener Adapter (10) für eine medizinische Vorrichtung, der folgende Komponenten umfaßt:
einen Körper (20) mit einer Längsachse (A), der ein proximales erstes Ende (22), das ein aufnehmendes Lüer-Anschlußteil (60) mit einer proximalen Fläche umfaßt, ein distales zweites Ende (24) und einen durchführenden Durchgang (26) mit einer Innenfläche (28) hat, wobei der Durchgang (26) eine Kammer (30) hat, die durch einen proximalen Absatz (32) und einen distalen Absatz (34) definiert wird, die von der Oberfläche (28) des Durchgangs (26) nach innen vorstehen, wobei sich die Kammer (30) zwischen dem ersten Ende (22) und dem zweiten Ende (24) befindet;
ein Ventil (40), das sich innerhalb der Kammer (30) befindet, um selektiv den Fluid-Fluß durch den Durchgang (26) zu verhindern und zu ermöglichen, wobei das Ventil (40) ein längliches elastisches Element (42) umfaßt, das eine Achse (A'), die im wesentlichen koaxial mit der Längsachse (A) des Körpers (20) ist, und ein distales Ende (44) und ein proximales Ende (46) hat, wobei das elastische Element (42) in Längsrichtung zwischen dem proximalen Absatz (32) und dem distalen Absatz (34) zusammengedrückt wird, wobei allein die Kompression des länglichen elastischen Elements (42) das Ventil (40) in eine normalerweise geschlossene Position vorspannt, um dadurch den Fluid-Fluß durch den Durchgang (26) zu verhindern; und
einen Stößel (50) auf dem länglichen elastischen Element (42), um das längliche elastische Element (42) selektiv in Längsrichtung weiter zusammenzudrücken, um dadurch das Ventil (40) zu öffnen und den Fluid-Fluß durch den Durchgang (26) zu ermöglichen, wobei der Stößel (50) einen proximal verlaufenden Abschnitt (55) innerhalb des aufnehmenden Lüer-Anschlußteils (60) hat, so daß dann, wenn eine Fluid-Handhabungsvorrichtung mit einem Lüer-Einsteckanschlußteil auf das proximale Ende (22) des Adapters (10) mit dem aufnehmenden Lüer-Anschlußteil (60) aufgesetzt wird, das Lüer-Einsteckanschlußteil mit dem Stößel (50) ineinandergreift, um dadurch das elastische Element (42) weiter zusammenzudrücken, so daß Fluid durch den Durchgang (26) fließen kann, wobei der proximal verlaufende Abschnitt (55) des Stößels eine Fläche (63) hat, die mit der proximalen Fläche des aufnehmenden Lüer-Anschlußteils (60) eine im wesentlichen durchgängige Fläche bildet, wodurch das aufnehmende Lüer-Anschlußteil (60) geschlossen wird, wenn sich das Ventil (40) in der normalerweise geschlossenen Position befindet;
dadurch gekennzeichnet, daß das längliche elastische Element (42) aus einem Elastomer gebildet wird, der distale Absatz (34) ein segmentierter distaler Absatz (34) ist, der zwischen den Segmenten (38) durchführende Öffnungen (36) hat,
das proximale Ende des elastischen Elements (42) mit dem proximalen Absatz (32) bei der Kompression des länglichen elastischen Elements (42) eine fluid-dichte Dichtung (48) bildet, wodurch das Ventil (40) in eine normalerweise geschlossene Position vorgespannt wird, um dadurch den Fluid-Fluß durch den Durchgang (26) zu verhindern, und
der Stößel (50) dem selektiven weiteren Zusammendrücken des länglichen elastischen Elements (42) in Längsrichtung dient, so daß das proximale Ende (46) des länglichen elastischen Elements (42) nicht mehr die fluid-dichte Dichtung mit dem proximalen Absatz (32) bildet.

2. Adapter (10) nach Anspruch 1, worin die Fläche des proximal verlaufenden Abschnitts (55) des Stößels (50) außerdem einen proximalen Vorsprung (65) einschließt, um den Fluid-Fluß aus dem Lüer-Einsteckanschlußteil in den Adapter (10) aus dem Lüer-Einsteckanschlußteil zu erleichtern.

3. Adapter (10) nach Anspruch 1, worin das distale Ende (24) des Körpers (20) Anbringungsmittel zur Anbringung des Adapters (10) an einer medizinischen Vorrichtung umfaßt.

4. Adapter (10) nach Anspruch 3, worin das Anbringungsmittel am distalen Ende des Körpers (20) ein Lüer-Einsteckanschlußteil umfaßt.

5. Adapter (10) nach Anspruch 3, worin das distale Ende des Körpers (20) einen fest angebrachten Katheterschlauch umfaßt.

6. Adapter (10) nach Anspruch 1, worin die Kammer (30) eine Vielzahl von Riefen (31) einschließt, um den Fluid-Fluß um das längliche elastische Element (42) zu erleichtern, wenn das Ventil (40) offen ist.

7. Adapter (10) nach Anspruch 1, worin das längliche elastische Element (42) außerdem einen Hohlraum (41) einschließt, der im wesentlichen koaxial mit der Achse (A) des Elements (42) verläuft, wobei der Hohlraum (41) an dem proximalen Ende (46) des Elements (42) ein offenes Ende (27) hat, um den Stößel (50) aufzunehmen, wobei der Hohlraum (41) eine Vielzahl von nach innen gerichteten Vorsprüngen (43) und Rippen (45) hat, wobei die Vielzahl proximale Vorsprünge und distale Rippen innerhalb des Hohlraums (41) umfaßt, wobei die proximalen Vorsprünge (43) dazu dienen, den Stößel (50) zu halten und, in Verbindung mit den distalen Rippen (45), dazu dienen, einen Kollapsbereich (53) zu bilden, wenn das elastische Element (42) in Längsrichtung weiter zusammengedrückt wird, um das Ventil (40) zu öffnen.

8. Adapter (10) nach Anspruch 1, worin die Vielzahl von Vorsprüngen (43) und Rippen (45) zwei diametral gegenüberliegende proximale Vorsprünge und zwei diametral gegenüberliegende distale Rippen umfaßt, wobei die proximalen Vorsprünge und die distalen Rippen so angeordnet sind, daß die diametralen Oppositionslinien orthogonal verlaufen.

9. Adapter (10c) nach Anspruch 1, worin der Körper (20c) zylindrisch ist und außerdem einen Seitenarm (120) einschließt, der einen durchführenden Durchgang (26c) hat, der in Fließverbindung mit der Kammer (30c) steht, wobei der Seitenarm (120) der Anbringung eines Schlauchs für den Fluid-Zugriff zu der Kammer (30c) dient.

10. Adapter (100) des 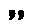 Y" -Typs, der zwei proximale Schenkel (104, 106) hat, die jeweils mit Ventil versehene Adapter (10b) nach Anspruch 1 umfassen, wobei die distalen zweiten Enden (108) des mit Ventil versehenen Adapters (10) in Fließverbindung stehen und mit einem gemeinsamen Lüer-Einsteckanschlußteil (114) verbunden sind.

11. Verteiler-Anschlußteil (200), das eine Vielzahl von mit Ventil versehenen Adaptern (10d) nach Anspruch 1 umfaßt, die zusammen fest angebracht sind.

## Revendications

1. Adaptateur à soupape (10) pour un dispositif médical, comprenant:
un corps (20) avec un axe longitudinal (A), comportant une première extrémité proximale (22) comprenant un raccord luer femelle (60) avec une surface proximale, une deuxième extrémité distale (24) et un passage (26) le traversant, avec une surface interne (28), ledit passage (26) comportant une chambre (30) définie par un épaulement proximal (32) et un épaulement distal (34), débordant vers l'intérieur à partir de ladite surface (28) dudit passage (26), ladite chambre (30) étant agencée entre ladite première extrémité (22) et ladite deuxième extrémité (24);
une soupape (40) contenue dans ladite chambre (30) pour arrêter et permettre sélectivement l' écoulement de fluide à travers ledit passage (26), ladite soupape (40) comprenant un élément élastique allongé (42) avec un axe (A') pratiquement coaxial audit axe longitudinal (A) dudit corps (20) et comportant une extrémité distale (44) et une extrémité proximale (46), ledit élément élastique (42) étant comprimé axialement entre ledit épaulement proximal (32) et ledit épaulement distal (34), ladite compression dudit élément élastique allongé (42) poussant seule ladite soupape (40) vers une position normalement fermée, arrêtant ainsi l'écoulement de fluide à travers ledit passage (26); et
un poussoir (50) sur ledit élément élastique allongé (42) pour comprimer sélectivement davantage ledit élément élastique allongé (42) dans une direction axiale, ouvrant ainsi ladite soupape (40) et permettant l'écoulement de fluide à travers ledit passage (26), ledit poussoir (50) comportant une partie à extension proximale (55) dans ledit raccord luer femelle (60), de sorte que lors du montage d'un dispositif de manipulation de fluide comportant un raccord luer mâle sur ladite extrémité proximale (22) dudit adaptateur (10) comportant ledit raccord luer femelle (60), le raccord luer mâle s'engage dans ledit poussoir (50) comprimant ainsi davantage ledit élément élastique (42), de sorte que le fluide peut s'écouler à travers ledit passage (26), ladite partie à extension proximale du poussoir (55) comportant une surface (63) formant une surface pratiquement continue avec la surface proximale dudit raccord luer femelle (60), fermant ainsi ledit raccord luer femelle (60) lorsque ladite soupape (40) se trouve dans ladite position normalement fermée;
caractérisé en ce que l'élément élastique allongé (42) est composé d'un élastomère, l'épaulement distal (34) étant un épaulement distal segmenté (34) comportant des ouvertures (36) le traversant entre les segments (38),
ladite extrémité proximale dudit élément élastique (42) forme un joint étanche aux fluides (48) avec ledit épaulement proximal (32) lors de ladite compression dudit élément élastique allongé (42), poussant ladite soupape (40) vers une position normalement fermée, arrêtant ainsi l'écoulement de fluide à travers ledit passage (26), et
ledit poussoir (50) sert à comprimer sélectivement davantage ledit élément élastique allongé (42) dans une direction axiale, de sorte que ladite extrémité proximale (46) dudit élément élastique allongé (42) ne forme plus ledit joint étanche aux fluides avec ledit épaulement proximal (32).

2. Adaptateur (10) selon la revendication 1, dans lequel ladite surface de ladite partie à extension proximale (55) dudit poussoir (50) englobe en outre une saillie proximale (65), destinée à faciliter l'écoulement du fluide à partir du raccord luer mâle dans ledit adaptateur (10) à partir du raccord luer mâle.

3. Adaptateur (10) selon la revendication 1, dans lequel ladite extrémité distale (24) dudit corps (20) comprend un moyen de fixation pour la fixation dudit adaptateur (10) à un dispositif médical.

4. Adaptateur (10) selon la revendication 3, dans lequel ledit moyen de fixation de l'extrémité distale dudit corps (20) comprend un raccord luer mâle.

5. Adaptateur (10) selon la revendication 3, dans lequel ladite extrémité distale dudit corps (20) comprend un tube de cathéter à fixation ferme.

6. Adaptateur (10) selon la revendication 1, dans lequel ladite chambre (30) englobe plusieurs canaux (31) pour faciliter l'écoulement de fluide autour dudit élément élastique allongé (42) lorsque ladite soupape (40) est ouverte.

7. Adaptateur (10) selon la revendication 1, dans lequel ledit élément élastique allongé (42) englobe en outre une cavité (41) pratiquement coaxiale audit axe (A) dudit élément (42) ladite cavité (41) comportant une extrémité ouverte (27) au niveau de ladite extrémité proximale (46) dudit élément (42) pour recevoir ledit poussoir (50), ladite cavité (41) comportant plusieurs saillies internes (43) et des nervures (45), lesdites plusieurs saillies et nervures comprenant des saillies proximales et des nervures distales dans ladite cavité (41), lesdites saillies proximales (43) servant à retenir ledit poussoir (50) et servant, en combinaison avec lesdites nervures distales (45), à former une région (53) d'affaissement lorsque ledit élément élastique (42) est comprimé davantage dans une direction axiale pour ouvrir ladite soupape (40).

8. Adaptateur (10) selon la revendication 1, dans lequel lesdites plusieurs saillies (43) et nervures (45) comprennent deux saillies proximales diamétralement opposées et deux nervures distales diamétralement opposées, lesdites saillies proximales et lesdites nervures distales étant agencées de sorte que lesdites lignes d'opposition diamétrales sont orthogonales.

9. Adaptateur (10) selon la revendication 1, dans lequel ledit corps (20c) est cylindrique et englobe en outre un bras latéral (120) comportant un passage (26c) le traversant, en connexion de fluide avec ladite chambre (30c), ledit bras latéral (120) étant destiné à monter un tube pour l'accès de fluide à ladite chambre (30c).

10. Adaptateur du type en "Y" (100) comportant deux bras proximaux (104, 106) comprenant chacun des adaptateurs à soupape (10b) selon la revendication 1, lesdites deuxièmes extrémités distales (108) dudit adaptateur à soupape (10) étant en connexion de fluide et étant reliées au niveau d'un raccord luer mâle commun (114).

11. Connecteur à rampe (200) comprenant plusieurs adaptateurs à soupape (10d) selon la revendication 1, fixés fermement les uns aux autres.
